# EUROPÄISCHE PATENTSCHRIFT

(11) **EP 1 455 612 B1**
(45) Veröffentlichungstag und Bekanntmachung des Hinweises auf die Patenterteilung: **15.11.2006**
(21) Anmeldenummer: 02796649.8
(22) Anmeldetag: 17.12.2002
(51) Int. Cl.: A41D 31/02

(54) **FLEXIBLE FLUSSIGKEITSDICHTE MATERIALBAHN**
FLEXIBLE FLUID-TIGHT WEB
BANDE DE MATERIAU FLEXIBLE ETANCHE AUX LIQUIDES

(30) Priorität: 17.12.2001 DE 20120392 U
(43) Veröffentlichungstag der Anmeldung: 15.09.2004
(73) Patentinhaber: Innovatec Microfibre Technology GmbH & Co.KG, 53840 Troisdorf (DE)
(72) Erfinder: KLEIN, Hans, Georg, 35764 Sinn (DE)
(74) Vertreter: Böck, Bernhard
(86) Internationale Anmeldenummer: PCT/EP2002/014352
(87) Internationale Veröffentlichungsnummer: WO 2003/052188

(56) Entgegenhaltungen:
- EP-A- 0 456 885
- EP-A- 1 013 291
- EP-A- 1 022 125
- EP-A- 1 088 537
- WO-A-97/30244
- WO-A-99/23140
- WO-A-99/33659
- WO-A-02/066086
- DE-A- 10 057 149
- FR-A- 2 762 781
- FR-A- 2 776 955
- NN-A-
- US-A- 5 417 789
- US-A- 5 908 412

## Beschreibung

Die Erfindung betrifft eine mehrlagige flüssigkeitsdichte Materialbahn für Schutzbekleidung und Hygieneartikel, nach dem Oberbegriff des Patentanspruchs 1. Die Erfindung betrifft ferner ein Verfahren zur Herstellung einer flexiblen Materialbahn nach Patentanspruch 16.

Materialien der eingangs genannten Art kommen beispielsweise, jedoch keineswegs ausschließlich, als Funktionsstoffe zur Herstellung von Spezialkleidung zum Schutz vor Chemikalien oder zum Einsatz in Reinräumen, zur Herstellung von Regenschutzbekleidung sowie als funktionelle Materialien, sogenannte Backsheets für Hygieneartikel, wie beispielsweise für Windeln, zum Einsatz. Derartige Materialien umfassen eine flüssigkeitsdichte Lage, die die Aufgabe des Schutzes gegen das Ein- bzw. Durchdringen von Fremdstoffen, Verunreinigungen, Chemikalien und anderen Flüssigkeiten, insbesondere Wasser, übernimmt.

Soweit diese Materialien Arbeitsschutzbekleidung betreffen, sind die Materialien gemäß europäischer Normen in verschiedene. Anforderungsklassen eingeteilt. In den Klassen 5 und 6 finden sich hauptsächlich ein- oder mehrlagige Mikrofaservliese. In den Klassen 3 und 4, die den Schutz vor flüssigen Chemikalien und anderen kritischen Flüssigkeiten betreffen, werden mit diversen Beschichtungen (z.B. PVC) ausgerüstete Stoffe bzw. Vliese eingesetzt.

Aus der US 5,908,412 ist ein beschichtetes Vliesmaterial für Wegwerf-Hygieneartikel bekannt. Das Vliesmaterial umfasst ein Trägervlies sowie eine Folie aus thermoplastischem Kunststoff, wobei die Folie im noch nicht erkalteten Zustand direkt aus einem Breitschlitzextruder auf das Trägervlies aufgebracht wird.

Ein Nachteil solcher Beschichtungen liegt zunächst einmal darin, dass derartige Beschichtungen insbesondere nicht wasserdampfdurchlässig sind. Dies hat beispielsweise bei Arbeitsschutzbekleidung zur Folge, dass Körperfeuchtigkeit nicht abgeführt, sondern gespeichert wird, worunter der Tragekomfort beträchtlich leidet. Außerdem lässt entweder die Haftung zwischen der Beschichtung und dem Vlies zu wünschen übrig, oder aber die Beschichtung führt zu einer unerwünschten Beeinträchtigung des textilen Charakters und der Elastizität des Trägermaterials.

Funktionsstoffe für die Sport- und Allwetterbekleidung, insbesondere wasserdichte diffusionsoffene bzw. wasserdampfdurchlässige Stoffe basieren auf komplexen und daher teuren, mehrlagigen Materialien, die in mehreren Arbeitsgängen gefertigt bzw. zusammengesetzt werden. Beispielsweise ist aus der WO 02/066086 ein dreilagiges atmungsaktives Laminat bekannt, wobei eine der Lagen durch eine aufwändig herzustellende und daher teure mikroporöse Membran aus Polyurethan gebildet ist. Dabei ist die mikroporöse Membran mit den weiteren Lagen mittels Heißschmelzkleber verbunden, was zusätzlichen Aufwand und zusätzliche Kosten verursacht.

Backsheets im Hygienebereich, also beispielsweise Windeln oder Inkontinenzeinlagen, sind in der Regel Zweilagen-Verbunde aus dampfdichten oder dampfoffenen PE-Filmen mit PP-Spinn- oder Stapelfaservliesen. Sie dienen als Flüssigkeitsschutz und in Verbindung mit weiteren, saugfähigen Materiallagen, beispielsweise mit einem Superabsorberpolymer (SAP), auch als Flüssigkeitsspeicher. Der Trend geht inzwischen zu den wasserdampfoffenen Produkten, mit denen der Tragekomfort wesentlich verbessert werden kann.

Hierzu kommen bei den aus dem Stand der Technik bekannten Backsheets mikroporöse PE-Filme zum Einsatz. Durch diese Materialien wird jedoch der gewünschte textile Charakter, bzw. der weiche Griff an der Materialoberfläche deutlich verschlechtert. Insbesondere weisen die aus dem Stand der Technik bekannten mikroporösen Filme, die zur Erzielung der Wasserdampfdurchlässigkeit mit hohen Füllstoffanteilen (CaCo₃) angereichert sind, diese nachteilige Eigenschaft auf.

Aus der FR 2 776 955 A1 ist ein zweilagiges Kompositmaterial mit Superabsorberpolymer bekannt, bei dem das Superabsorberpolymer-Pulver mittels Heißschmelzkleber mit einem Trägervlies verbunden wird. Dieses Kompositmaterial dient dem Flüssigkeitsschutz von Kabelummantelungen, indem beim Eindringen von Flüssigkeit durch die Kabelummantelung das mit dem Trägervlies verbundene Superabsorberpolymer expandiert und dem eindringenden Wasser damit den weiteren Weg versperrt. Das aus dieser Druckschrift bekannte Verbundmaterial eignet sich mangels einer atmungsaktiven Membran sowie mangels fester Verankerung der Superabsorberpolymer Partikel in der Materialbahn jedoch nicht für den Einsatz bei hochwertiger Schutzbekleidung oder für entsprechende Hygieneartikel.

Aus der Druckschrift WO 99/33659 A1 ist eine mehrlagige Materialbahn bekannt, bei der ein flächiger poröser Träger mit einem hydrophilen und daher wasserdampfdurchlässigen Copolymer beschichtet ist. Die WO 99/33659 A1 liefert auch Hinweise darauf, die wasserdampfdurchlässige Copolymer-Schicht unmittelbar im schmelzflüssigen Zustand auf den flächigen porösen Träger aufzubringen. Mangels Vorhandensein von Absorptivstoffen eignet sich die Materialbahn gemäß WO 99/33659 A1 jedoch nicht für Hygieneprodukte, bei denen die Aufnahme großer Mengen von Flüssigkeit im Vordergrund steht.

Mit diesem Hintergrund ist es die Aufgabe der vorliegenden Erfindung, eine atmungsaktive Materialbahn mit einer stoff- bzw. haftschlüssig auf einem porösen Träger angeordneten Polymerlage, sowie ein Verfahren zu deren Herstellung zu schaffen, mit der die genannten Nachteile überwunden werden und die zudem äußerst kostengünstig gefertigt werden kann. Dabei soll die Materialbahn gleichzeitig durch enthaltenes pulverförmiges Superabsorberpolymer hochabsorptionsfähig sein. Überdies soll das Superabsorberpolymer insbesondere so fest in die Materialbahn eingebettet sein, dass der Materialverbund unter allen Umständen, auch nach der Aufnahme großer Mengen von Flüssigkeiten, in sich stabil bleibt und sogar bei Verletzungen einer Deckschicht praktisch kein Austritt von Superabsorberpolymer-Gel erfolgen kann. Ferner soll sich die Materialbahn zur Herstellung von Hygieneprodukten mit besonders guten textilen Eigenschaften eignen.

Diese Aufgabe wird durch eine Materialbahn mit den Merkmalen des Patentanspruchs 1 bzw. durch ein Herstellungsverfahren für eine Materialbahn mit den Merkmalen des Patentanspruchs 16 gelöst.

Bevorzugte Ausführungsformen sind Gegenstand der Unteransprüche.

Die Materialbahn gemäß der vorliegenden Erfindung ist in zunächst an sich bekannter Weise als zwei- oder mehrlagiger Verbund aufgebaut und enthält pulverförmiges Superabsorberpolymer. Dabei umfasst die Materialbahn einen flächigen porösen Träger, beispielsweise einen Trägerstoff oder ein Trägervlies, sowie eine auf dem Träger angeordnete flüssigkeitsdichte Lage in Form eines unmittelbar auf den porösen Träger aufgebrachten diffusionsoffenen Heißschmelz-Klebefilms, der wie eine atmungsaktive Membran wirkt. Durch den direkt auf den porösen Träger aufgebrachten Heißschmelz-Klebefilm kann auf eine zusätzliche Membran oder Folie verzichtet werden, ohne dass dadurch Funktionseinbußen bezüglich der Flüssigkeitsdichtigkeit oder Atmungsaktivität der Materialbahn gemacht werden müssten.

Da der Heißschmelz-Klebefilm äußerst dünn gehalten werden kann, werden zudem die mechanische Eigenschaften sowie der "Look-and-Feel" des Trägermaterials praktisch nicht beeinflusst oder verändert, was einerseits einer optimalen Verarbeitungsfähigkeit bei der Herstellung von Textilien oder Hygieneartikeln dient und andererseits den Tragekomfort derartiger Textilien oder Hygieneartikel erheblich verbessert. Außerdem erfolgt der Auftrag des Heißschmelzklebers auf das Trägermaterial im heißen Zustand, so dass zusammen mit den hervorragenden Hafteigenschaften des Heißschmelzklebers eine flächige, innige Verbindung zwischen dem Heißschmelz-Klebefilm und dem Trägermaterial erzielt wird.

Erfindungsgemäß zeichnet sich die Materialbahn jedoch dadurch aus, dass das Superabsorberpolymer in den Heißschmelzkleber eingebettet ist. Dank der erfindungsgemäß in den diffusionsoffenen Heißschmelzkleber-Film eingebetteten Superabsorberpolymer-Partikel wird eine Materialbahn erhalten, die einerseits äußerst kostengünstig und rationell zu fertigen ist, andererseits jedoch gute Absorptionseigenschaften mit einer äußerst festen Verankerung des Superabsorberpolymer-Pulvers in der Materialbahn und damit eine extrem hohe Auslaufsicherheit, selbst im Fall von Beschädigungen der Materialbahn, aufweist.

Versuche haben gezeigt, dass Materialbahnen mit optimalen mechanischen Eigenschaften und mit optimaler Wasserdichtigkeit beispielsweise dann erhalten werden, wenn, wie dies eine weitere bevorzugte Ausführungsform der Erfindung vorsieht, als Material für den Heißschmelz-Klebefilm Äthylvinylacetate, Polyamide, Polyvinylalkohole oder Polyurethane eingesetzt werden. Gemäß einer weiteren Ausführungsform weist das Material für den Heißschmelz-Klebefilm hydrophile Bestandteile und eine damit verbundene Oberflächenspannung von ≤ 20mN/m auf.

Insbesondere die hydrophilen Bestandteile sorgen dafür, dass der Heißschmelz-Klebefilm die gewünschten Durchlasseigenschaften für Wassermoleküle, d. h. für Wasserdampf erhält, wobei jedoch gleichzeitig die Dichtigkeit gegenüber Flüssigkeiten erhalten bleibt. Es hat sich gezeigt, dass sich Materialbahnen mit besonders vorteilhaften Eigenschaften erzeugen lassen, wenn für die hydrophilen Bestandteile des Heißschmelz-Klebefilms Elastomere auf Basis von Polytetramethylenoxid, Comonomere von Polypropylenoxid und Polyehtylenoxid oder hydrophile Polymerweichmacher verwendet werden. Vor allem letztere zeichnen sich dadurch aus, dass sie besonders kostengünstig sind.

Ein weiterer Vorteil der Erfindung liegt darin, dass sich durch das Prinzip der Ausbildung der flüssigkeitsdichten Lage durch Aufbringen von Heißschmelzkleber auf das Trägermaterial flüssigkeitsdichte Lagen in jeder beliebigen Stärke erzeugen lassen. Bevorzugt liegt das Auftragsgewicht der Heißschmelzkleberbeschichtung je nach Anwendungsfall jedoch zwischen 5 bis 50 g/m².

Bei den Trägermaterialien handelt es sich bevorzugt um poröse, luftdurchlässige, flächige Körper, beispielsweise um Vliese. Besonders bevorzugt liegen die Trägerschichten in Form von Polypropylen-, Polyäthylenterephtalat- oder Polyäthylen- Spinn-, Nadel-, oder Mikrofaservliesen (Meltblown) vor.

Überraschend hat sich gezeigt, dass damit nach dem Aufkaschieren des Heißschmelz-Klebefilms noch Wasserdampfdurchlässigkeiten von ≥ 200 g/m²×d erreicht werden. Das Flächengewicht des Trägers liegt dabei bevorzugt im Bereich von 10 bis 150 g/m², bei der Verwendung von Spinnvlies aus Polypropylen bzw. niedrig- oder hochdichtem Polyäthylen bevorzugt im Bereich von 10 bis 60 g/m².

Ein weiterer besonderer Vorteil der erfindungsgemäß durch einen Heißschmelz-Klebefilm gebildeten flüssigkeitsdichten Lage liegt darin, dass die so gebildete flüssigkeitsdichte Lage nicht nur einen innigen Verbund mit dem Grundmaterial eingeht, sondern darüber hinaus auch noch zur Aufgabe der Kaschierung bzw. Verklebung mit weiteren Materiallagen herangezogen werden kann. Mit diesem Hintergrund ist es gemäß einer weiteren Ausführungsform der Erfindung vorgesehen, dass auf dem Heißschmelz-Klebefilm zusätzlich eine Deckschichtlage angeordnet ist. Dabei kann es sich beispielsweise um ein einfaches Deckvlies oder aber auch um eine mehrlagige Deckschicht handeln.

Gemäß einer weiteren Ausführungsform der Erfindung kann darüber hinaus das Deckvlies bzw. die Oberfläche einer auf dem Heißschmelz-Klebefilm angeordneten mehrlagigen Deckschicht zudem durch öl- oder fetthaltige Avivagen besonders hautfreundlich ausgerüstet sein. Dies ist insbesondere vorteilhaft für Hygieneartikel bei dem dort vorhandenen direkten Hautkontakt.

Um insbesondere bei großflächigen Produkten, wie beispielsweise bei Inkontinenzeinlagen, oder auch bei besonders hohen mechanischen Beanspruchungen, die Eigenstabilität und Festigkeit des Materialverbundes zu erhöhen, ist es gemäß einer weiteren Ausführungsform der Erfindung vorgesehen, dass in oder auf dem Heißschmelz-Klebefilm ein Armierungsgewebe bzw. ein Armierungsgitter angeordnet wird. Durch die Einbettung in den Hotmeltfilm bzw. durch das Anhaften am Hotmeltfilm wird eine besonders gute Krafteinleitung und Kraftverteilung bei mechanischer Beanspruchung des Verbundes erzielt.

Gemäß einer weiteren Ausführungsform der Erfindung zeichnet sich die Materialbahn dadurch aus, dass die Materialbahn in den Heißschmelzkleber eingebettetes oder auf der Oberfläche des Heißschmelzklebers angeordnetes, pulverförmiges Superabsorberpolymer (SAP), beispielsweise auf Basis eines gelbildenden Polyacrylsäureesters, umfasst. Auf diese Weise lassen sich besonders einfach, zuverlässig und kostengünstig insbesondere hochabsorptionsfähige Verbundmaterialien für Hygieneprodukte erzeugen. Dabei liegt ein besonderer Vorteil der Einbettung bzw. Anhaftung des SAP-Pulvers am Heißschmelzkleber darin, dass der Materialverbund auch nach der Aufnahme großer Mengen von Flüssigkeiten in sich stabil bleibt und sogar bei Verletzungen einer Deckschicht praktisch kein Austritt von SAP-Gel erfolgt.

Insbesondere bei Hygieneanwendungen, wie beispielsweise für Windeln, bei denen es auf ein extrem hohes Feuchtigkeitsaufnahmevermögen ankommt, ist es vorteilhaft, wie dies eine weitere bevorzugte Ausführungsform der Erfindung vorsieht, wenn das Superabsorberpolymer mit einem hydrophilen Mikrofaservlies, insbesondere mit einem sogenannten Meltblown abgedeckt bzw. umgeben ist. Das so unmittelbar an das SAP-Pulver angrenzende hydrophile Mikrofaservlies sorgt beim Flüssigkeitseintritt aufgrund seiner starken Dochtwirkung für eine schnelle und großflächigen Verteilung der aufgenommenen Flüssigkeit, so dass bei Flüssigkeitseintritt ein möglichst großer Flächenbereich des SAP-Pulvers benetzt bzw. aktiviert wird.

Gemäß einer weiteren bevorzugten Ausführungsform der Erfindung ist das Superabsorberpolymer mit einem Verbund aus einer Lage hydrophilem Spinnvlies, beispielsweise aus Polypropylen oder Polyäthylen mit einem bevorzugten Flächengewicht von 8 bis 50 g/m², und einer Lage hydrophilem Meltblown, beispielsweise aus Polypropylen, mit einem bevorzugten Flächengewicht von 10 bis 100 g/m² abgedeckt. Dabei übernimmt, wie oben beschrieben, das unmittelbar an das SAP-Pulver angrenzende Meltblown wiederum die Aufgabe der raschen und großflächigen Verteilung eindringender Flüssigkeit. Die mit dem Meltblown verbundene Spinnvlies-Decklage erfüllt insbesondere mechanische und dekorative Aufgaben und eignet sich außerdem besonders gut für einen direkten Hautkontakt.

Anstelle der Meltblown-Lage kann auch eine textile Beflockung zum Einsatz kommen, die entweder auf den Hotmeltfilm bzw. auf das SAP-Pulver, oder aber auf der Rückseite des hydrophilen Spinnvlieses aufgebracht sein kann. Der Einsatz einer textilen Beflockung ist insbesondere vorteilhaft im Hinblick auf die schnellere Herstellbarkeit, die innigere Verbindung mit dem Hotmeltfilm bzw. mit dem SAP, und kann darüber hinaus beispielsweise ohne weiteres auch auf eine unregelmäßige bzw. zerklüftete Oberfläche aufgetragen werden.

Besonders vorteilhaft ist es ferner, wenn der Heißschmelz-Klebefilm bei seinem Auftrag auf die Trägerschicht in die Zwischenräume des zerklüfteten Trägervlieses eingedrückt wird bzw. sich in diese Zwischenräume einsenkt. Aufgrund der dabei auftretenden Oberflächenvergrößerung bei gleichzeitiger Dickenreduzierung des Heißsehmelz-Klebefilms wird- die für den Feuchtigkeitstransport wirksame Fläche erheblich vergrößert. Dadurch kann die Wasserdampfdurchlässigkeit eines dergestalt erzeugten Verbundmaterials noch einmal um den Faktor drei bis fünf gesteigert werden und liegt dann, bei 23° C und 0%./.85%, höher als 200 g/m²×d.

Ein weiterer Vorteil des Eindringens von Heißschmelzkleber bis in die Hohlräume des Trägervlieses liegt darin, dass die einzelnen Fasern des Trägervlieses dadurch miteinander verklebt werden; was die mechanische Stabilität des Trägers und damit auch des gesamten Verbundes erheblich erhöhen kann.

Der Heiß-Schmelz-Klebefilm kann beispielsweise auch zwischen zwei wasser- und darinpfdurchlässigen Trägermaterialien, z.B. textilen Vliesen liegen. Ein Vlies bildet dann den Träger und das andere das Deckvlies.

Ferner betrifft die Erfindung ein Verfahren zur Herstellung einer flexiblen, atmungsaktiven und flüssigkeitsdichten Materialbahn für Schutzbekleidung oder Hygieneartikel. Dabei umfasst die Materialbahn eine flächige poröse Trägerschicht, pulverförmiges Superabsorberpolymer sowie eine auf der Trägerschicht angeordnete flüssigkeitsdichte Lage Das Verfahren umfasst den Verfahrensschritt des Aufbringens eines noch schmelzflüssigen Polymerfilms, bestehend aus diffusionsoffenem Heißschmelzkleber, auf den porösen Träger.

Erfindungsgemäß zeichnet sich das Verfahren jedoch dadurch aus, dass das Superabsorberpolymer-Pulver unmittelbar auf die noch flüssige Schmelze des Heißschmelzklebers aufgestreut wird, wonach die Decklage auf das Superabsorberpolymer-Pulver und auf den Heißschmelzkleber aufgebracht wird und die Lagen der Materialbahn mittels Kalandrierung dergestalt miteinander verklebt werden, dass das Superabsorberpolymer-Pulver in den Heißschmelzkleber eingebettet wird.

Mit dem erfindungsgeinäßen Verfahren lassen sich wasserdichte, jedoch atmungsaktive mehrlagige Materialbahnen besonders kostengünstig fertigen, wobei gleichzeitig eine außergewöhnlich hohe Qualität und Stabilität der so hergestellten Materialbahnen erzielt wird. Dies liegt insbesondere darin begründet, dass der Hotmeltfilm bei dem erfindungsgemäßen Verfahren mehrere vollkommen verschiedene Funktionen gleichzeitig erfüllen kann,

So bildet der Hotmeltfilm einerseits zunächst eine Barriere für Flüssigkeiten und ermöglicht so, für die Trägerschicht anstelle von Kunststofffolien die besonders hautfreundlichen und griffsympathischen Mikrofaservliese zu verwenden. Andererseits ermöglicht der Hotmeltfilm aufgrund seiner geringen Dicke und aufgrund seines diffusionsoffenen Materialcharakters den Durchgang von Wasserdampf durch die flexible Materialbahn. Diese Eigenschaften prädestinieren die so hergestellte Materialbahn für den Einsatz bei anspruchsvollen Hygieneanwendungen und für hochwertige Schutzbekleidung.

Zusätzlich dient der Hotmeltfilm dank des eingebetteten Superabsorberpolymers der unverrückbaren Verankerung des Superabsorberpolymer-Pulvers in der Materialbahn. Hierdurch wird eine Materialbahn erhalten, die zunächst einmal große Mengen von Flüssigkeit absorbieren kann. Im Vergleich zum Stand der Technik werden auf diese Weise jedoch mechanisch besonders stabile und belastbare, hochabsorptive sowie wasserdichte und atmungsaktive Materialbahnen erzeugt, die auch noch nach der Absorption großer Mengen von Flüssigkeiten auslaufsicher sind: Selbst im Fall eventueller Materialverletzungen behält die nach dem erfindungsgemäßen Verfahren hergestellte Materialbahn ihre Form weitestgehend bei und verliert kein Superabsorberpolymer-Gel, wie dies beim Stand der Technik der Fall ist.

Schließlich erfüllt der Hotmeltfilm auch noch die zusätzliche Aufgabe der Verklebung der einzelnen Materiallagen untereinander, wobei durch das Eindringen des Heißschmelzklebers zwischen die an der Oberfläche der einzelnen Materiallagen gelegenen Fasern sogar noch eine weitere Stabilisierung des Verbundes erzielt werden kann.

Gemäß einer bevorzugten Ausführungsform des erfindungsgemäßen Verfahrens ist die im Verfahrensschritt c) aufgebrachte Decklage ein Verbund aus einem Spinnvlies und einem Meltblown (SM-Verbund). Hierdurch werden besonders hochabsorptionsfähige Materialbahnen erhalten, da das Meltblown aufgrund seiner überaus starken Kapillar-bzw. Dochtwirkung eingedrungene Flüssigkeiten sehr rasch über eine große Fläche verteilen kann, wodurch ein möglichst großer Flächenbereich des Superabsorberpolymer-Pulvers benetzt bzw. aktiviert wird. Das Spinnvlies übernimmt dabei die Aufgabe der mechanischen Stabilisierung und Begrenzung des Meltblown an der Materialoberfläche.

Anstelle die Decklage im Verfahrensschritt c) als fertigen Verbund aus Meltblown und Spinnvlies zuzuführen, kann die Aufgabe des Meltblown auch durch eine vor dem abschließenden Aufbringen des Spinnvlieses aufgebrachte textile Beflockung ersetzt werden. Dabei kann die textile Beflockung entweder auf dem Hotmeltfilm bzw. auf der Superabsorberpölymer-Lage oder aber auf der Rückseite des Spinnvlieses angebracht werden. Auf diese Weise können die Absorptionseigenschaften des herzustellenden Verbundmaterials durch entsprechende Anpassung der Parameter der textilen Beflockung noch in weiten Grenzen eingestellt werden, ohne dass das Verfahren auf andere zuzuführende Materialarten umzustellen wäre.

Um den Zusammenhalt der einzelnen Materiallagen des Verbundes weiter zu verbessern, ist es gemäß einer weiteren Ausführungsform des erfindungsgemäßen Verfahrens vorgesehen, das unmittelbar vor dem Aufbringen der Decklage im Verfahrensschritt c) ein Hotmelt-Sprühauftrag auf der Innenseite der Decklage erfolgt. Auf diese Weise wird die Haftung der auf die Superabsorberpolymer-Schicht aufgebrachten Decklage weiter verbessert, was zu mechanisch besonders hoch belastbaren Materialverbunden führt.

Es ist für das Wesen der Erfindung nicht bestimmend, wie die konstruktive bzw. betriebliche Umsetzung des erfindungsgemäßen Verfahrens erfolgt. So kann das erfindungsgemäße Verfahren beispielsweise diskontinuierlich bzw. Off-Line durchgeführt werden. Gemäß einer bevorzugten Ausführungsform der Erfindung werden jedoch die Verfahrensschritte a) bis c) im kontinuierlichen Inline-Verfahren durchgeführt. Auf diese Weise lässt sich ein besonders hoher Durchsatz bei gleichzeitig hoher Reproduzierliarkeit der Qualitätsparameter des Materialverbundes erzielen.

Um mechanisch besonders hoch belastbare Materialverbunde zu erhalten, ist es gemäß einer weiteren Ausführungsform der Erfindung vorgesehen, dass nach dem Verfahrensschritt a) in den Hotmeltfilm ein Armierungsgewebe bzw. Armierungsgitter eingelegt bzw. auf den Hotmeltfilm aufgelegt wird. Das Armierungsgewebe bzw. -gitter verbindet sich dabei innig mit der noch heißen Schmelze des Hotmeltfilms. Auf diese Weise wird eine großflächige Krafteinleitung in den Materialverbund und Kraftverteilung im Materialverbund ermöglicht, und es lassen sich dank des Zusammenwirkens von Armierung und Verankerung durch den Heißschmelzkleber extrem belastungsfähige Materialverbünde herstellen.

Im Folgenden wird die Erfindung anhand lediglich Ausführungsbeispiele darstellender Zeichnungen näher erläutert.

Es zeigt:
- **Fig. 1**: in nicht maßstabsgetreuer, schematischer seitlicher Schnittdarstellung die prinzipielle Funktionsweise eines flüssigkeitsdichten absorptionsfähigen Materialverbundes des gemäß dem Stand der Technik;
- **Fig. 2**: in einer **Fig. 1** entsprechenden Darstellung Aufbau und Funktionsweise eines Materialverbundes gemäß der vorliegenden Erfindung;
- **Fig. 3**: in einer **Fig. 1** und **2** entsprechenden Darstellung den prinzipiellen Aufbau eines flüssigkeitsdichten Verbundes aus Trägerschicht und Membran gemäß dem Stand der Technik ;
- **Fig. 4**: in einer **Fig. 1** bis **3** entsprechenden Darstellung den prinzipiellen Aufbau eines flüssigkeitsdichten Verbundes gemäß der vorliegenden Erfindung;
- **Fig. 5**: in einer **Fig. 1** bis **5** entsprechenden Darstellung das erfindungsgemäße Prinzip der Oberflächenvergrößerung des Hotmeltfilms;
- **Fig. 6**: in einer **Fig. 1** bis **4** entsprechenden Darstellung ein Ausführungsbeispiel eines Mehrlagenverbundes;
- **Fig. 7**: in einer **Fig. 1** bis **6** entsprechenden Darstellung den Membranbereich des Mehrlagenverbunds gemäß Fig. 6 in vergrößerter Darstellung;
- **Fig. 8**: in einer **Fig. 1** bis **7** entsprechenden Darstellung das erfindungsgemäBe Prinzip der gegenseitigen Verankerung verschiedener Materiallagen;
- **Fig. 9**: in einer **Fig. 1** bis **8** entsprechenden Darstellung den prinzipiellen Aufbau eines Verbundes mit eingelagertem Superabsorberpolymer; und
- **Fig. 10**: in einer **Fig. 1** bis **9** entsprechenden Darstellung den Aufbau eines Verbundes mit aufgestreutem Superabsorberpolymer;

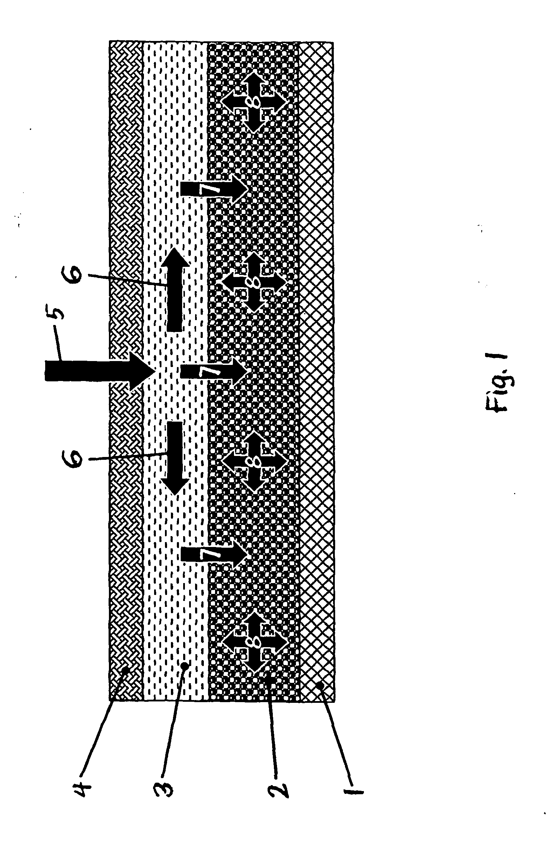

In **Fig. 1** ist in schematischer Darstellung der Querschnitt eines flüssigkeitsdichten absorptionsfähigen Materialverbundes dargestellt, wie er im Prinzip aus dem Stand der Technik bekannt ist.

Man erkennt zunächst die zeichnungsbezogen unten liegende Trägerschicht 1, die gemäß dem Stand der Technik im allgemeinen aus einer ggf. mikroporösen Polyäthylenfolie besteht. Unmittelbar auf der Trägerschicht 1 ist der Absorptionskern 2 angeordnet, der aus einem hochabsorptionsfähigen Material, beispielsweise aus einer superabsorberpolymerhaltigen Materiallage besteht. Oberhalb des Absorptionskerns 2 wiederum befindet sich die Erfassungsschicht 3, die der raschen Absorption, der Flüssigkeitsaufnahme in den Mehrlagenverbund sowie der Flüssigkeitsverteilung innerhalb der Ebene des Mehrlagenverbundes dient. Die Erfassungsschicht 3 besteht im allgemeinen aus einem Stapelfaservlies bzw. Meltblown. Ferner umfasst der Materialverbund die Deckschicht 4, die im allgemeinen in Form eines flüssigkeitsdurchlässigen Spinnvlieses vorliegt.

Man erkennt in **Fig. 1** außerdem das Prinzip der Flüssigkeitsaufnahme in den Materialverbund sowie der Flüssigkeitsverteilung und -speicherung im Materialverbund. Der mit Bezugsziffer 5 bezeichnete Pfeil steht für eine auf die Deckschicht 4 aufgebrachte Flüssigkeitsmenge, die aufgrund des offenporigen Charakters der Deckschicht 4 sowie aufgrund der starken Kapillar- bzw. Dochtwirkung der Erfassungsschicht 3 sofort durch die Deckschicht 4 hindurch gesaugt und zunächst von der Erfassungsschicht 3 aufgesogen wird. Ebenso aufgrund der starken Dochtwirkung wird die aufgesogene Flüssigkeitsmenge 5 innerhalb der Fläche der Erfassungsschicht 3 zeichnungsbezogen horizontal verteilt, was die mit Bezugsziffer 6 bezeichneten Pfeile verdeutlichen.

Im Anschluss an die horizontale Verteilung 6 der eingedrungenen Flüssigkeit 5 wird diese von der Erfassungsschicht 3 an die Absorptionsschicht 2 weitergegeben, was die mit Bezugsziffer 7 bezeichneten Pfeile andeuten. Innerhalb der Absorptionsschicht 2 wird die Flüssigkeitsmenge 5 durch Aktivierung, Expansion und Gelbildung des in der Absorptionsschicht 2 enthaltenen Superabsorberpolymers schließlich aufgenommen und als Gel gebunden, was die eine Expansion andeutenden Pfeile 8 versinnbildlichen.

Wie eingangs beschrieben, weist dieser aus dem Stand der Technik bekannte Aufbau absorptionsfähiger Mehrlagenverbunde diverse Nachteile auf, von denen hier insbesondere der mangelnde Tragekomfort bzw. die mangelnde Grifffreundlichkeit der die Trägerschicht darstellenden Polyäthylenfolie 1, der komplizierte Aufbau und insbesondere der mangelnde Zusammenhalt der einzelnen Schichten und die mangelnde Verankerung des Superabsorberpolymers innerhalb der Absorptionsschicht 2 nochmals genannt werden sollen.

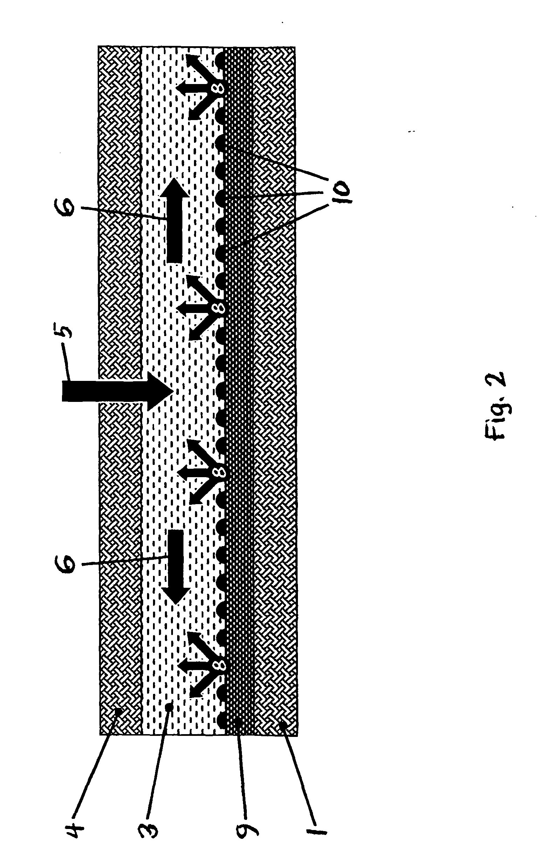

Diese Nachteile werden durch den in **Fig. 2** dargestellten, eine Ausführungsform der vorliegenden Erfindung verkörpernden Mehrlagenverbund eliminiert. Der Mehrlagenverbund gemäß **Fig. 2** besitzt ebenfalls eine Trägerschicht 1. Im Unterschied zum Stand der Technik liegt die Trägerschicht 1 gemäß **Fig. 2** jedoch, ebenso wie die Deckschicht 4, in Form eines äußerst luftdurchlässigen Faservlieses vor, was insbesondere den Tragekomfort und den "Look-and-Feel" dergestalt aufgebauter Materialverbunde entscheidend verbessert.

Die erforderliche Flüssigkeitsdichtigkeit wird bei dem Materialverbund gemäß **Fig. 2** durch eine unmittelbar auf der Trägerschicht 1 aufgebrachte, flüssigkeitsdichte Membran 9 gewährleistet, die erfindungsgemäß in Form einer Heißschmelzkleber-Schicht 9 vorliegt.

Unmittelbar auf die Hotmelt-Schicht 9 wiederum ist das Superabsorberpolymer in Form von fein verteiltem Pulver 10 aufgebracht. Damit ist insbesondere der Vorteil verbunden, dass das Superabsorberpolymer 10 über den Hotmeltfilm 9 mit der Trägerschicht 1 verbunden bzw. räumlich verankert ist, was ganz beträchtlich zur mechanischen Stabilität, Auslaufsicherheit und damit Gebrauchstauglichkeit eines so aufgebauten Materialverbunds beiträgt.

Zeichnungsbezogen oberhalb des Hotmeltfilms 9 bzw. des Superabsorberpolymers 10 ist wieder eine Erfassungsschicht 3 angeordnet, die der schnellen Aufsaugung und flächigen Verteilung 6 einer auf den Materialverbund aufgebrachten Flüssigkeitsmenge 5 dient. Anschließend wird die Flüssigkeitsmenge 5 durch Aktivierung, Expansion und Gelbildung der Superabsorberpolymerkügelchen 10 im Materialverbund gemäß **Fig. 2** gebunden, was die eine Expansion des Superabsorberpolymers 10 versinnbildlichenden Pfeile 8 darstellen.

Die Decklage des Materialverbunds gemäß **Fig. 2** kann vorzugsweise aus einem sogenannten SM-Verbund bestehen, der ein aus Spinnvlies 4 und Meltblown 3 bestehendes Halbzeug darstellt. In diesem Zusammenhang kommt ein weiterer entscheidender Vorteil des erfindungsgemäß aufgebauten Materialverbundes gemäß **Fig. 2** zum Tragen, der, wie bereits beschrieben, mit der Mehrfachfunktion des Hotmeltfilms 9 zusammenhängt. Denn der Hotmeltfilm 9 dient sowohl der Erzeugung der flüssigkeitsdichten Lage 9 des Materialverbunds, als auch der Verankerung von Trägerschicht 1, Superabsorberpolymer 10 und SM-Decklage 3, 4 untereinander, wobei letztere Verankerungswirkung der verschiedenen Lagen des Materialverbunds untereinander zu mechanisch besonders belastbaren und damit besonders hochwertigen Materialverbunden führt.

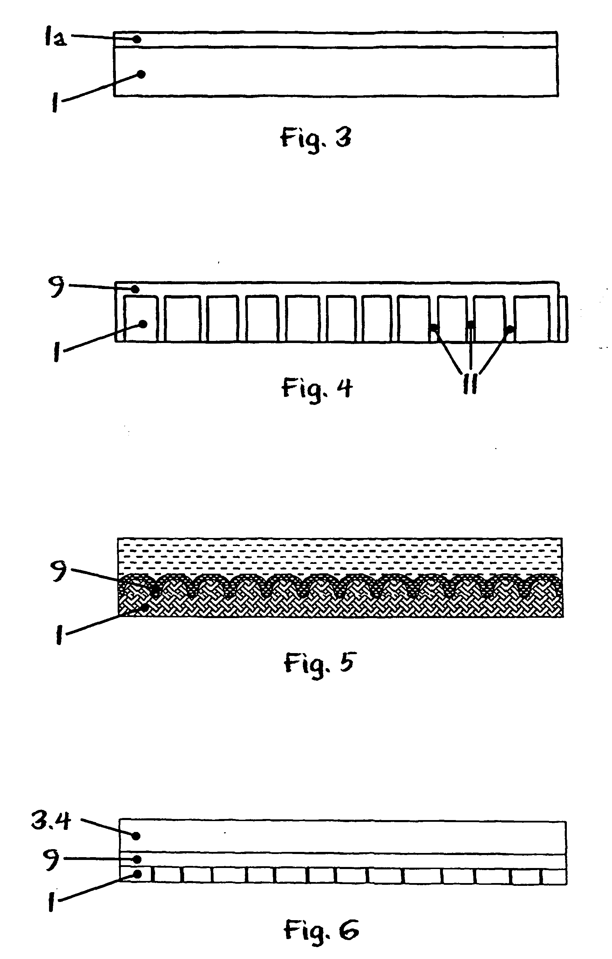

Die **Fig. 3** und **4** verdeutlichen insbesondere den Unterschied zwischen einem flüssigkeitsdichten Verbund aus Trägerschicht 1 und Membran 1a gemäß dem Stand der Technik (**Fig. 3**) und einem ebensolchen Materialverbund aus Trägerschicht 1 und Hotmeltfilm 9 gemäß der vorliegenden Erfindung (**Fig. 4**). Dabei zeigt **Fig. 4** in nicht maßstabsgetreuer Vergrößerung einen Ausschnitt aus dem Grenzbereich zwischen Trägerschicht 1 und Hotmeltfilm 9. Man erkennt, dass der Hotmeltfilm 9 aufgrund seines Auftrags auf die Trägerschicht 1 im heißen flüssigen Zustand bei Bezugsziffer 11 in die Poren der aus einem Faservlieses bestehenden Trägerschicht 1 eindringt, wodurch eine besonders gute Verankerung zwischen dem die Membran bildenden Hotmeltfilm 9 und der Trägerschicht 1 entsteht.

Ein weiterer mit dem Eindringen bzw. Einsenken des heißflüssig aufgebrachten Hotmeltfilms 9 in die Poren des die Trägerschicht bildenden Faservlieses 1 liegt darin, dass dadurch, wie in **Fig. 5** schematisch dargestellt, die Oberfläche des Hotmeltfilms 9 beträchtlich vergrößert wird, wobei sich gleichzeitig die Dicke des Hotmeltfilms 9 entsprechend verringert. Dies ist insbesondere dort von Bedeutung, wo hydrophile, also wasserdampfdurchlässige Materialien für die Erzeugung der Hotmeltschicht 9 eingesetzt werden, da auf diese Weise die wünschenswerte, besonders hohe Wasserdampfdurchlässigkeit erzielt wird.

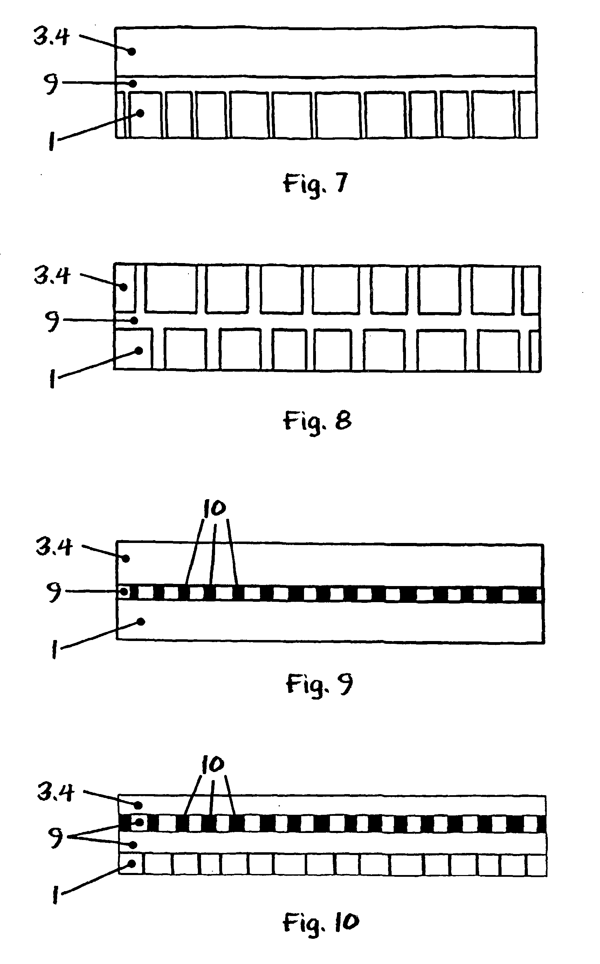

In **Fig. 6** ist wiederum in höchst schematischer Darstellung ein Ausführungsbeispiel für einen Mehrlagenverbund dargestellt. Man erkennt die Trägerschicht 1 mit dem schematisch angedeuteten, in die Trägerschicht 1 eingesenkten Hotmeltfilm 9. Außerdem umfasst der Mehrlagenverbund gemäß **Fig. 6** eine beispielsweise aus einem SM-Verbund bestehende Decklage 3, 4.

**Fig. 7** und **Fig. 8** zeigen jeweils den Bereich der aus dem Hotmeltfilm 9 gebildeten Membran und die daran angrenzenden Oberflächen der Trägerschicht 1 bzw. der aus einem SM-Verbund bestehenden Decklage 3, 4. Man erkennt, dass bei der Ausführungsform gemäß **Fig. 7** aufgrund einer entsprechenden Verfahrensführung der Hotmeltfilm 9 besonders innig mit der Trägerschicht 1 verankert ist, während bei der Ausführungsform gemäß **Fig. 8**, ebenfalls durch entsprechende Verfahrensführung bei der Herstellung des Verbundes darüber hinaus auch eine dementsprechend feste Verankerung des Hotmeltfilms 9 mit dem SM-Verbund 3, 4 erfolgt ist.

In den **Fig. 9** und **10** schließlich sind wiederum höchst schematisch zwei Ausführungsformen für einen Materialverbund dargestellt, der sich zusätzlich durch in Verbund enthaltenes Superabsorberpolymer-Pulver 10 auszeichnet. Dabei ist das Superabsorberpolymer-Pulver 10 bei der Ausführungsform gemäß **Fig. 9** im wesentlichen vollständig im Hotmeltfilm 9 eingebettet, während es bei der Ausführungsform gemäß **Fig. 10** auf den noch heißen Hotmeltfilm 9 lediglich aufgestreut wurde und daher an dessen Oberfläche angeordnet ist.

Im Ergebnis wird somit erkennbar, dass sich dank der erfindungsgemäßen Materialbahn und dank des erfindungsgemäßen Verfahrens zur Herstellung von Materialbahnen wichtige Eigenschaften wie Wasserdampfdurchlässigkeit, Flüssigkeitsdichtigkeit, mechanische Beanspruchbarkeit und Tragekomfort von Funktions- und Schutzkleidung bzw. von hochabsorptionsfähigen Hygieneartikeln, insbesondere von Windeln, entscheidend verbessern lassen, wobei sich die Herstellungskosten der Materialbahnen dank der Erfindung trotz verbesserter Materialeigenschaften nicht nennenswert erhöhen, sondern sogar reduziert werden können.

## Patentansprüche

1. Flexible, flüssigkeitsdichte Materialbahn für Schutzbekleidung oder Hygieneartikel, aus einem einen flächigen porösen Träger (1), beispielsweise ein Trägervlies, umfassenden zwei- oder mehrlagigen Verbund, wobei eine flüssigkeitsdichte Polymerlage (9) form- und haftschlüssig auf dem Träger (1) angeordnet ist, wobei die flüssigkeitsdichte Polymerlage (9) ein Film ist, der aus diffusionsoffenem Heißschmelzkleber besteht und wobei die Materialbahn pulverförmiges Superabsorberpolymer umfasst,
**dadurch gekennzeichnet,**
**dass** das Superabsorberpolymer (10) in den Heißschmelzkleber (9) eingebettet ist.

2. Materialbahn nach Anspruch 1,
**dadurch gekennzeichnet,**
**dass** der Heißschmelzkleber (9) aus EVA, PA, PVAL, PUR, mit oder ohne hydrophile Bestandteile, besteht.

3. Materialbahn nach Anspruch 1 oder 2,
**dadurch gekennzeichnet,**
**dass** der hydrophile Bestandteil ein Elastomer auf Basis Polytetramethylenoxid, Comonomer von Polypropylenoxid und Polyehtylenoxid oder ein hydrophiler Polymerweichmacher ist.

4. Materialbahn nach einem der Ansprüche 1 bis 3,
**dadurch gekennzeichnet,**
**dass** das Auftragsgewicht des Heißschmelzklebers (9) zwischen 5 bis 50 g/m² liegt.

5. Materialbahn nach einem der Ansprüche 1 bis 4,
**dadurch gekennzeichnet,**
**dass** der poröse Träger (1) ein PP-, PET- oder PE- Spinn- oder Nadelvlies, ein wasserverfestigtes Vlies oder ein Mikrofaservlies ist.

6. Materialbahn nach einem der Ansprüche 1 bis 5,
**dadurch gekennzeichnet,**
**dass** der poröse Träger (1) ein textiles Vlies mit einem Flächengewicht von 10 bis 150 g/m² ist.

7. Materialbahn nach einem der Ansprüche 1 bis 6,
**dadurch gekennzeichnet,**
**dass** der poröse Träger (1) ein Spinnvlies aus PP, HDPE oder LDPE mit einem Flächengewicht von 10 bis 60 g/m² ist.

8. Materialbahn nach einem der Ansprüche 1 bis 7,
**gekennzeichnet durch**
eine auf dem Heißschmelzkleber (9) angeordnete Decklage (3, 4).

9. Materialbahn nach einem der Ansprüche 1 bis 8,
**dadurch gekennzeichnet,**
**dass** die die Decklage (3, 4) durch öl- oder fetthaltige Avivagen hautfreundlich ausgerüstet ist.

10. Materialbahn nach einem der Ansprüche 1 bis 9,
**gekennzeichnet durch**
ein in oder auf dem Heißschmelzkleber (9) angeordnetes Armierungsgewebe bzw. Armierungsgitter.

11. Materialbahn nach einem der Ansprüche 1 bis 10,
**dadurch gekennzeichnet,**
**dass** das Superabsorberpolymer (10) mit einem hydrophilen Mikrofaservlies (4) abgedeckt ist.

12. Materialbahn nach einem der Ansprüche 1 bis 11,
**dadurch gekennzeichnet,**
**dass** das Superabsorberpolymer (10) mit einem SM-Verbund (3, 4) aus hydrophilem Spinnvlies (PP, HDPE, LDPE) (4) mit einem bevorzugten Flächengewicht von 8 bis 50g/m² und aus hydrophilem Meltblown (PP) (3) mit einem bevorzugten Flächengewicht von 10 bis 100 g/m² abgedeckt ist

13. Materialbahn nach Anspruch 12,
**dadurch gekennzeichnet,**
**dass** das Meltblown (3) durch eine textile Beflockung ersetzt ist.

14. Materialbahn nach einem der Ansprüche 1 bis 13,
**dadurch gekennzeichnet,**
**dass** der Heißschmelzkleber (9) in Zwischenräume bzw. Hohlräume (11) des Trägervlieses und/oder des Deckvlieses eingesenkt ist.

15. Materialbahn nach einem der Ansprüche 1 bis 14, **gekennzeichnet durch** eine
eine bei 23° C und 0%./.85% gegebene Wasserdampfdurchlässigkeit größer als 200 g/m²×d.

16. Verfahren zur Herstellung einer pulverförmiges Superabsorberpolymer enthaltenden flexiblen flüssigkeitsdichten Materialbahn für Schutzbekleidung oder Hygieneartikel, die Materialbahn bestehend aus zwei oder mehr verbundenen Lagen sowie umfassend einen flächigen porösen Träger (1) mit einer auf dem Träger (1) angeordneten flüssigkeitsdichten Lage (9), das Verfahren umfassend den Verfahrensschritt:
a) Aufbringen eines noch schmelzflüssigen Polymerfilms (9) auf den porösen Träger (1), wobei der Polymerfilm (9) aus diffusionsoffenem Heißschmelzkleber besteht;
**dadurch gekennzeichnet,**
**dass** sich dem im Verfahrensschritt a) erfolgten Aufbringen des Polymerfilms (9) der folgende Verfahrensschritt anschließt:
b) Aufstreuen von Superabsorberpolymer-Pulver (10) unmittelbar auf die noch flüssige Schmelze des Heißschmelzklebers (9);
c) Aufbringen einer Decklage (3, 4); und
d) Verklebung der Lagen durch Kalandrierung dergestalt, dass das Superabsorberpolymer-Pulver in den Heißschmelzkleber eingebettet wird.

17. Verfahren nach Anspruch 16,
**dadurch gekennzeichnet,**
**dass** die Decklage (3, 4) im Verfahrensschritt c) ein SM-Verbund aus Spinnvlies (4) und Meltblown (3) ist.

18. Verfahren nach Anspruch 17,
**dadurch gekennzeichnet,**
**dass** das Meltblown (3) durch eine textile Beflockung ersetzt ist.

19. Verfahren nach einem der Ansprüche 16 bis 18,
**dadurch gekennzeichnet,**
**dass** vor dem Aufbringen der Decklage (3, 4) im Verfahrensschritt c) ein Heißschmelzkleber-Sprühauftrag auf die Innenseite der Decklage (3, 4) erfolgt.

20. Verfahren nach einem der Ansprüche 16 bis 19,
**dadurch gekennzeichnet,**
**dass** die Verfahrensschritte a) bis c) im kontinuierlichen Inline-Verfahren durchgeführt werden.

21. Verfahren nach einem der Ansprüche 16 bis 20,
**dadurch gekennzeichnet,**
**dass** nach Verfahrensschritt a) in den oder auf den Heißschmelzkleber (9) ein Armierungsgewebe bzw. Armierungsgitter aufgebracht wird.

## Claims

1. A flexible fluid-tight material web for protective clothing or personal care articles, out of a two or multi-layer composite fleece backing comprising a porous sheet backing (1), for example afleece a carrier fleece, where a fluid-tight polymer layer (9) is arranged on said backing (1) in a form-locking and tight-fitting manner, where said fluid-tight polymer layer (9) is a film consisting of a hot melt adhesive open to diffusion and where said material web comprising a powdery super absorber polymer, **characterized in that** said super absorber polymer (10) is embedded in said hot melt adhesive (9).

2. The material web as set forth in claim 1, **characterized in that** said hot melt adhesive (9) consists of EVA, PA, PVAL, PUR with or without hydrophilic components.

3. The material web as set forth in claim 1 or 2, **characterized in that** said hydrophilic component is an elastomer based on polytetramethylene oxide, a comonomer of polypropylene oxide and polyethylene oxide or a hydrophilic polymer plasticizer.

4. The material web as set forth in any of the claims 1 to 3, **characterized in that** the coating weight of said hot melt adhesive (9) is in the range 5 to 50 g/m2.

5. The material web as set forth in any of the claims 1 to 4, **characterized in that** said said porous backing (1) is a PP, PET or PE spunbond or needle-punched fleece, a water consolidated fleece or a microfiber fleece.

6. The material web as set forth in any of the claims 1 to 5, **characterized in that** said porous backing (1) is a textile fleece having a weight in the range 10 to 150 g/m2.

7. The material web as set forth in any of the claims 1 to 6, **characterized in that** said porous backing (1) is a spunbond fleece of PP, HDPE or LDPE having a weight in the range 10 to 60 g/ m2.

8. The material web as set forth in any of the claims 1 to 7, **characterized by** a cover layer (3,4) disposed on said hot melt adhesive (9).

9. The material web as set forth in any of the claims 1 to 8, **characterized in that** said cover layer (3, 4) is engineered kind to the skin by oily or fatty avivages.

10. The material web as set forth in any of the claims 1 to 9, **characterized by** a reinforcement fabric or reinforcement mesh disposed in or on said hot melt adhesive (9).

11. The material web as set forth in any of the claims 1 to 10, **characterized in that** said super absorber polymer (10) is covered by a hydrophilic microfiber fleece (4).

12. The material web as set forth in any of the claims 1 to 11, **characterized in that** said super absorber polymer (10) is covered by a SM composite (3,4) of hydrophilic spunbond fleece (PP, HDPE, LDPE) (4) having a weight preferably in the range 8 to 50 g/m2 and of a hydrophilic melt blown (PP) (3) having a weight preferably in the range 10 to 100 g/ m2.

13. The material web as set forth in claim 12, **characterized in that** said melt blown (3) is replaced by a textile flaking.

14. The material web as set forth in any of the claims 1 to 13, **characterized in that** said hot melt adhesive (9) is submerged into interspaces or cavities (11) of said backing fleece and/or covering fleece.

15. The material web as set forth in any of the claims 1 to 14, **characterized by** a water vapor permeability higher than 200 g/m2 x d at 23°C and 0% ./.85%.

16. A method of producing a flexible fluid-tight material web, containing a powdery super absorber polymer, for protective clothing or personal care articles, where said material web comprises two or more joined layers and including a porous sheet backing (1) including a fluid-tight layer (9) arranged on said backing (1), said method comprising the steps of
a) applying a still molten polymer film (9) onto said porous backing (1), where said polymer film (9) consists of a hot melt adhesive open to diffusion;
**characterized in that**
the applying of said polymer film (9) done in step a) is followed by the following method step of:
b) powdering super absorber polymer powder (10) directly onto said still hot melt of said hot melt adhesive (9) ;
c) applying a cover layer (3, 4); and
d) bonding said layers by calandering such that said super absorber polymer powder (10) is embedded in said hot melt adhesive.

17. The method as set forth in claim 16, **characterized in that** said cover layer (3,4) in the method step c) is an SM composite of spunbond fleece (4) and melt blown (3).

18. The method as set forth in claim 17, **characterized in that** said melt blown (3) is replaced by a textile flaking.

19. The method as set forth in any of the claims 16 to 18, **characterized in that** prior to applying said cover layer (3,4) in said method step c), a hot melt spray coating is made on the inside of said cover layer (3,4).

20. The method as set forth in any of the claims 16 to 19, **characterized in that** steps a) to c) are implemented in a continuos inline method.

21. The method as set forth in any of the claims 16 to 20, **characterized in that** after method step a) a reinforcement fabric or reinforcement mesh is applied within or on said hot melt adhesive (9).

## Revendications

1. Bande de matériau flexible, imperméable pour liquides, destinée pour l'équipement de protection ou les articles d'hygiène consistant en une combinaison formée de deux ou plusieurs couches, qui comprend un support étendu poreux (1), comme par exemple une couverture-support, où une couche imperméable pour liquides sur base de polymère (9) est disposée en forme identique et adhérente sur le support (1), où la couche imperméable pour liquides sur base de polymère (9) est un film, formé d'un adhésif se fondant à chaud et capable de diffusion et où la bande de matériau contient un polymère avec superabsorption dans l'état de poudre, **caractérisée en ce que** le polymère avec superabsorption (10) est incorporé dans l'adhésif se fondant à chaud (9).

2. Bande de matériau selon la revendication 1, **caractérisée en ce que** l'adhésif se fondant à chaud (9) est formé d' EVA, PA, PVAL, PUR avec ou sans des composantes hydrophiles.

3. Bande de matériau selon la revendication 1 ou 2, **caractérisée en ce que** la composante hydrophile est un élastomère sur base d'oxyde de polytétraméthylène, comonomère avec l'oxyde de polypropylène et l'oxyde de polyéthylène ou un plastifiant de polymère hydrophile.

4. Bande de matériau selon l'une des revendications 1 à 3, **caractérisée en ce que** le poids de déposition de l'adhésif se fondant à chaud (9) est compris entre 5 et 50 g/m².

5. Bande de matériau selon l'une des revendications 1 à 4, **caractérisée en ce que** le support poreux (1) est une couche PP, PET ou PEN, de type tissé ou avec des aiguilles, une couche imperméable pour l'eau ou une couche de microfibres.

6. Bande de matériau selon l'une des revendications 1 à 5, **caractérisée en ce que** le support poreux (1) est une couche textile avec un poids superficielle de 10 à 150 g/m².

7. Bande de matériau selon l'une des revendications 1 à 6, **caractérisée en ce que** le support poreux (1) est une couche tissée de PP, HDPE ou LDPE avec un poids superficiel de 10 à 60 g/m².

8. Bande de matériau selon l'une des revendications 1 à 7, **caractérisée par** une couche de recouvrement (3, 4) déposée sur l'adhésif se fondant à chaud (9).

9. Bande de matériau selon l'une des revendications 1 à 8, **caractérisée en ce que** la couche de recouvrement (3, 4) est préparée agréablement pour la peau par addition d'huiles et de graisses.

10. Bande de matériau selon l'une des revendications 1 à 9, **caractérisée par** un tissu de renforcement, respectivement, un filet de renforcement déposé dans ou sur l'adhésif se fondant à chaud (9).

11. Bande de matériau selon l'une des revendications 1 à 10, **caractérisée en ce que** le polymère avec absorption (10) est recouvert avec une couche hydrophile de microfibres (4).

12. Bande de matériau selon l'une des revendications 1 à 11, **caractérisée en ce que** le polymère avec superabsorption (10) est recouvert avec une combinaison SM (3, 4) d'une couche hydrophile tissée (PP, HDPE, LDPE) (4) avec un poids sur surface préféré de 8 à 50 g/m² et d'une couche hydrophile fondue soufflée (3) avec un poids sur surface préféré de 10 à 100 g/m².

13. Bande de matériau selon la 12, **caractérisée en ce que** la couche fondue soufflée (3) est remplacée avec une couche de fibres textiles.

14. Bande de matériau selon l'une des revendications 1 à 13, **caractérisée en ce que** l'adhésif se fondant à chaud (9) est fixé dans les intervalles et les creux (11) de la couche de support et/ou de la couche de recouvrement.

15. Bande de matériau selon l'une des revendications 1 à 14, **caractérisée par** une perméabilité à vapeurs d'eau donnée à 23 °C et 0% / 85% plus que 200 g/m²xd.

16. Procédé pour la production d'une bande de matériau flexible et imperméable pour liquides, contenant le polymère avec superabsorption en forme de poudre destiné pour l'équipement de protection ou des articles d'hygiène, où la bande de matériau este composée de deux ou plusieurs couches combinées et contenant un support étendu poreux (1) avec une couche imperméable pour liquides (9) disposée sur le support (1), le procédé comprenant l'étape:
a) la déposition d'un film polymère dans l'état fondu (9) sur le support poreux (1), où le film polymère (9) est formé de l'adhésif se fondant à chaud capable de diffusion; **caractérisé en ce que** l'étape de déposition du film polymère (9) de l'étape a) est succédée de la suivante étape:
b) la déposition de poudre de polymère avec superabsorption (10) directement sur la masse fondue encore fluide de l'adhésif se fondant à chaud (9);
c) la déposition d'une couche de recouvrement (3, 4); et
d) le collage des couches par calandrage de sorte que la poudre de polymère avec superabsorption soit incorporée dans l'adhésif se fondant à chaud.

17. Procédé selon la revendication 16, **caractérisé en ce que** la couche de recouvrement (3, 4) de l'étape c) est une combinaison SM de couche tissée (4) et de couche fondue soufflée (3).

18. Procédé selon la revendication 17, **caractérisé en ce que** la couche fondue soufflée (3) est remplacée avec une couche textile.

19. Procédé selon l'une des revendications 16 à 18, **caractérisé en ce qu'**avant de la déposition de la couche de recouvrement (3, 4) dans l'étape c) a lieu une déposition par pulvérisation de l'adhésif se fondant à chaud sur la surface intérieure de la couche de recouvrement (3, 4).

20. Procédé selon l'une des revendications 16 à 19, **caractérisé en ce que** les étapes a) à c) se déroulent dans un procédé continu en ligne.

21. Procédé selon l'une des revendications 16 à 20, **caractérisé en ce qu'**après l'étape a) on dépose un tissu de renforcement, respectivement un filet de renforcement dans ou sur l'adhésif se fondant à chaud (9).
